# EUROPEAN PATENT SPECIFICATION

(11) **EP 2 513 023 B1**
(45) Date of publication and mention of the grant of the patent: **13.11.2019**
(21) Application number: 10842581.0
(22) Date of filing: 16.12.2010
(51) Int. Cl.: C07C 21/18, C08J 9/04, C09K 3/30, C11D 3/24, C10M 131/04

(54) **COMPOSITIONS AND USES OF CIS-1,1,1,4,4,4-HEXAFLUORO-2-BUTENE**
ZUSAMMENSETZUNGEN AUS CIS-1,1,1,4,4,4-HEXAFLUOR-2-BUTEN UND IHRE VERWENDUNGEN
COMPOSITIONS ET UTILISATIONS DE CIS-1,1,1,4,4,4-HEXAFLUOROBUT-2-ÈNE

(30) Priority: 15.12.2010 US 968506; 16.12.2009 US 287033 P
(43) Date of publication of application: 24.10.2012
(60) Divisional application: 17196370.5
(73) Proprietor: Honeywell International Inc., Morris Plains, NJ 07950 (US)
(72) Inventor: HULSE, Ryan, Getzville New York 14068 (US); ZYHOWSKI, Gary John, Lancaster New York 14086 (US); HOFMAN, Bjiorn, Morris Plains New Jersey 07950 (US); WILLIAMS, Dave, Morristown New Jersey 07962-2245 (US); KNOPECK, Gary, Morristown New Jersey 07962 (US); RICHARD, Robert G., Morristown New Jersey 07962 (US); BASU, Rajat, Morristown New Jersey 07962 (US); SINGH, Rajiv Ratna, Getzville New York 14068 (US)
(74) Representative: Crooks, Elizabeth Caroline
(86) International application number: PCT/US2010/060646
(87) International publication number: WO 2011/084553

(56) References cited:
- WO-A1-2008/121776
- WO-A1-2008/154612
- WO-A1-2009/032983
- WO-A2-2008/134061
- WO-A2-2010/062888
- US-A1- 2006 243 945
- US-A1- 2007 010 592
- US-A1- 2007 108 403

## Description

### FIELD OF THE INVENTION

This invention relates to compositions containing the compound cis-1,1,1,4,4,4-hexafluoro-2-butene (Z-HFO-1336mzzm), which has the following structure:

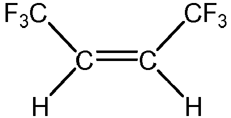

### BACKGROUND OF THE INVENTION

WO 2008/134061 discloses azeotropic or azeotrope-like compositions comprising cis-1,1,1,4,4,4-hexafluoro-2-butene.

WO 2009/032983 discloses azeotropic or azeotrope-like compositions comprising E-1,1,1,4,4,5,5,5-octafluoro-2-pentene with methyl formate, n-pentane, 2-methylbutane, 1,1,1,3,3-pentafluorobutane, trans-1,2-dichloroethylene, 1,1,1,3,3-pentafluoropropane, dimethoxymethane, cyclopentane or Z-1,1,1,4,4,4-hexafluoro-2-butene.

US2007/108403 discloses compositions for use in refrigeration, air-conditioning or heat pump systems wherein the composition comprises at least one fluoroolefin.

WO 2010/062888 discloses an absorption cycle system comprising a refrigerant.

The compositions of the present invention are part of a continued search for the next generation of low global warming potential materials. Such materials must have low environmental impact, as measured by ultra-low global warming potential and zero ozone depletion potential.

### SUMMARY OF THE INVENTION

This invention relates to compositions having utility in numerous applications, and in particular, uses for compositions containing the compound cis-1,1,1,4,4,4-hexafluoro-2-butene (Z-HFO-1336mzzm), which has the following structure:

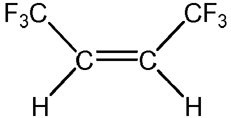

Embodiments of the present invention comprise the compound Z-HFO-1336mzzm in combination with HFO-1233zd described in detail herein below. Preferably, mixtures containing the compound Z-HFO-1336mzzm are non-azeotropic.

It should be noted that it would be common and expected for a product designated as Z-HFO-1336mzzm to include a minor percentage, for example about 0.5 wt % up to about 5 wt % of other components, including particularly E-HFO-1336mzzm. When used herein, the term "consisting essentially of Z-HFO-1336mzzm" is intended to generally include such compositions. The terms "consist of' and "consisting of' as used herein, do not include such other components. All of the embodiments of the invention described herein may, if desired, be obtained in a substantially purified form, such that these embodiments preferably consist of only the actual components designated, other than minor (e.g., ppm) impurities.

The compositions described herein may be used as blowing agents.

### DETAILED DESCRIPTION OF PREFERRED EMBODIMENTS

The compositions of the present invention all include the compound Z-HFO-1336mzzm. Certain embodiments of the invention, particularly those employed as blowing agent compositions or foamable compositions, can optionally include other ingredients, some of which are described in detail below.

In addition to the compound Z-HFO-1336mzzm, described herein are compositions comprising, or consisting essentially of, at least one additional fluoroalkene containing three carbon atoms, and at least one carbon-carbon double bond. The fluoroalkene compounds of the present invention are sometimes referred to herein for the purpose of convenience as hydrofluoro-olefins or "HFOs" if they contain at least one hydrogen.

Applicants have developed several compositions which include as an essential component the compound Z-HFO-1336mzzm and HFO-1233zd. In such compositions, the amount of the compound Z-HFO-1336mzzm may vary widely, including in all cases constituting the balance of the composition after all other components in composition are accounted for.

The amount of the compound Z-HFO-1336mzzm in the composition may be in accordance with the following ranges: from about 1 wt % to about 99 wt %; from about 30 wt % to about 99 wt %; from about 50 wt % to about 99 wt %; from about 75 wt % to about 99 wt %; from about 85 wt % to about 99 wt %; from about 20 wt % to about 80 wt %; from about 90 wt % to about 99 wt %; from about 95 wt % to about 99 wt %; from about 1 wt % to about 20 wt %; from about 1 wt % to about 40 wt %; from about 1 wt % to about 50 wt %; from about 5 wt % to about 20 wt %; from about 5 wt % to about 40 wt %; from about 5 wt % to about 60 wt %; from about 10 wt % to about 80 wt %; from about 10 wt % to about 90 wt %; from about 20 wt % to about 80 wt %; from about 20 wt % to about 90 wt %.

The preferred compositions of the present invention are environmentally acceptable and do not to contribute to the depletion of the earth's stratospheric ozone layer. The compounds and compositions of the present invention have no substantial ozone depletion potential (ODP), preferably an ODP of not greater than about 0.5 and even more preferably an ODP of not greater than about 0.25, most preferably an ODP of not greater than about 0.1; and/or a global warming potential (GWP) of not greater than about 150, and even more preferably, a GWP of not greater than about 50.

As used herein, ODP is defined in the "Scientific Assessment of Ozone Depletion, 2002," a report of the World Meteorological association. As used herein, GWP is defined relative to that of carbon dioxide and over a 100 year time horizon, and defined in the same reference as for the ODP mentioned above.

Preferred compositions of this type are described below in Table 1 (with all percentages being in percent by weight and being understood to be proceeded by the word "about").

**Table 1 - Blend Compositions**

| Compound mixed with Z-HFO-1336mzzm | Range wt % |
|---|---|
| HFO-1233zd | 30 to 70 |

### USES OF THE COMPOSITIONS

As described above, the compositions of the present invention may be used in a wide variety of applications as substitutes for CFCs and for compositions containing less desirable HCFCs. For example, the present compositions are useful as blowing agents. This use will be discussed in greater detail below.

### BLOWING AGENTS

Thus, the present invention includes methods and systems which include using Z-HFO-1336mzzm as a blowing agent, optionally with one or more optional additional compounds which include, but are not limited to, other compounds which also act as blowing agents (hereinafter referred to for convenience but not by way of limitation as co-blowing agents), surfactants, polyols, catalysts, flame retardants, polymer modifiers, colorants, dyes, solubility enhancers, rheology modifiers, plasticizing agents, fillers, nucleating agents, viscosity reduction agents, vapor pressure modifiers, stabilizers, and the like. Preferred blends for blowing agents used for foams, especially spray foams and panel foams include blends of Z-HFO-1336mzzm with 1233zd.

For this use, the amount of the compound Z-HFO-1336mzzm in the composition of the invention may be in accordance with the following ranges: from about 1 wt % to about 99 wt %; from about 30 wt % to about 99 wt %; from about 50 wt % to about 99 wt %; from about 75 wt % to about 99 wt %; from about 85 wt % to about 99 wt %; from about 20 wt % to about 80 wt %; from about 90 wt % to about 99 wt %; from about 95 wt % to about 99 wt %; from about 1 wt % to about 20 wt %; from about 1 wt % to about 40 wt %; from about 1 wt % to about 50 wt %; from about 5 wt % to about 20 wt %; from about 5 wt % to about 40 wt %; from about 5 wt % to about 60 wt %; from about 10 wt % to about 80 wt %; from about 10 wt % to about 90 wt %; from about 20 wt % to about 80 wt %; from about 20 wt % to about 90 wt %. Another range of amounts is shown in Table 1, and that amount is likewise applicable for this use of the composition of the invention.

In certain preferred embodiments, dispersing agents, cell stabilizers, surfactants and other additives may also be incorporated into the blowing agent compositions of the present invention. Certain surfactants are optionally but preferably added to serve as cell stabilizers. Some representative materials are sold under the names of DC-193, B-8404, and L-5340 which are, generally, polysiloxane polyoxyalkylene block co-polymers such as those disclosed in U.S. Pat. Nos. 2,834,748, 2,917,480, and 2,846,458. Other optional additives for the blowing agent mixture may include flame retardants such as tri(2-chloroethyl)phosphate, tri(2-chloropropyl)phosphate, tri(2,3-dibromopropyl)-phosphate, tri(1,3-dichloropropyl)phosphate, diammonium phosphate, various halogenated aromatic compounds, antimony oxide, aluminum trihydrate, polyvinyl chloride, and the like. With respect to nucleating agents, all known compounds and materials having nucleating functionality are available for use in the present invention, including particularly talc.

Of course other compounds and/or components that modulate a particular property of the compositions (such as cost for example) may also be included in the present compositions, and the presence of all such compounds and components is within the broad scope of the invention.

The co-blowing agent in accordance with the present invention can comprise a physical blowing agent, a chemical blowing agent (which preferably in certain embodiments comprises water) or a blowing agent having a combination of physical and chemical blowing agent properties.

Although it is contemplated that a wide range of co-blowing agents may be used in accordance with the present invention, in certain embodiments it is preferred that the blowing agent compositions of the present invention include one or more HFCs as co-blowing agents, more preferably one or more C1-C4 HFCs, and/or one or more hydrocarbons, more preferably C4-C6 hydrocarbons. For example, with respect to HFCs, the present blowing agent compositions may include one or more of difluoromethane (HFC-32), fluoroethane (HFC-161), difluoroethane (HFC-152), trifluoroethane (HFC-143), tetrafluoroethane (HFC-134), pentafluoroethane (HFC-125), pentafluoropropane (HFC-245), hexafluoropropane (HFC-236), heptafluoropropane (HFC-227ea), pentafluorobutane (HFC-365mfc), hexafluorobutane (HFC-356) and all isomers of all such HFC's.

With respect to hydrocarbons, the present blowing agent compositions may include in certain preferred embodiments, for example, iso, normal and/or cyclopentane for thermoset foams and butane or isobutane for thermoplastic foams. Of course other materials, such as water, CO2, CFCs (such as trichlorofluoromethane (CFC-11) and dichlorodifluoromethane (CFC-12)), hydrochlorocarbons (HCCs such as dichloro-ethylene (preferably trans-1,2-dichloroethylene), ethyl chloride and chloropropane), HCFCs, C1-C5 alcohols (such as, for example, ethanol and/or propanol and/or butanol), C1-C4 aldehydes, C1-C4 ketones, C1-C4 ethers (including ethers (such as dimethyl ether and diethyl ether), diethers (such as dimethoxy methane and diethoxy methane)), and methyl formate including combinations of any of these may be included, although such components are contemplated to be not preferred in many embodiments due to negative environmental impact.

In certain embodiments, one or more of the following HFC isomers are preferred for use as co-blowing agents in the compositions of the present invention:
1,1,1,2,2-pentafluoroethane (HFC-125)
1,1,2,2-tetrafluoroethane (HFC-134)
1,1,1,2-tetrafluoroethane (HFC-134a)
1,1-difluoroethane (HFC-152a)
1,1,1,2,3,3,3-heptafluoropropane (HFC-227ea)
1,1,1,3,3,3-hexafluoropropane (HFC-236fa)
1,1,1,3,3-pentafluoropropane (HFC-245fa) and
1,1,1,3,3-pentafluorobutane (HFC-365mfc).

The relative amount of any of the above noted additional co-blowing agents, as well as any additional components which may be included in present compositions, can vary widely within the general broad scope of the present invention according to the particular application for the composition, and all such relative amounts are considered to be within the scope hereof.

In certain embodiments it is preferred that the blowing agent composition of the present invention comprise at least one co-blowing agent and an amount of Z-HFO-1336mzzm sufficient to produce a blowing agent composition which is overall nonflammable.

The blowing agent compositions of the present invention may include the compound Z-HFO-1336mzzm in widely ranging amounts. It is generally preferred, however, that for preferred compositions for use as blowing agents in accordance with the present invention, Z-HFO-1336mzzm is present in an amount that is at least about 1% by weight, more preferably at least about 5% by weight, and even more preferably at least about 15% by weight, of the composition.

In certain preferred embodiments, the blowing agent comprises at least about 50% by weight of the present blowing agent compound(s), and in certain embodiments the blowing agent consists essentially of Z-HFO-1336mzzm. In this regard it is noted that the use of one or more co-blowing agents is consistent with the novel and basic features of the present invention. For example, it is contemplated that water will be used as either a co-blowing or in combination with other co-blowing agents (such as, for example, pentane, particularly cyclopentane) in a large number of embodiments.

In certain preferred embodiments, the blowing agent composition comprises from about 30% to about 95% by weight of Z-HFO-1336mzzm and from about 5% to about 90% by weight, more preferably from about 5% to about 65% by weight of co-blowing agent.

In preferred embodiments in which the co-blowing agent comprises H2O, the composition comprises H2O in an amount of from about 5% by weight to about 50% by weight of the total blowing agent composition, more preferably from about 10% by weight to about 40% by weight, and even more preferably of from about 10% to about 20% by weight of the total blowing agent.

In preferred embodiments in which the co-blowing agent comprises CO2, the composition comprises CO2 in an amount of from about 5% by weight to about 60% by weight of the total blowing agent composition, more preferably from about 20% by weight to about 50% by weight, and even more preferably of from about 40% to about 50% by weight of the total blowing agent.

In preferred embodiments in which the co-blowing agent comprises alcohols, (preferably C2, C3 and/or C4 alcohols), the composition comprises alcohol in an amount of from about 5% by weight to about 40% by weight of the total blowing agent composition, more preferably from about 10% by weight to about 40% by weight, and even more preferably of from about 15% to about 25% by weight of the total blowing agent.

For compositions which include HFC co-blowing agents, the HFC co-blowing agent (preferably C2, C3, C4 and/or C5 HFC), and even more preferably difluoromethane (HFC-152a) (HFC-152a being particularly preferred for extruded thermoplastics) and/or pentafluoropropane (HFC-245)), is preferably present in the composition in amounts of from of from about 5% by weight to about 80% by weight of the total blowing agent composition, more preferably from about 10% by weight to about 75% by weight, and even more preferably of from about 25% to about 75% by weight of the total blowing agent. Furthermore, in such embodiments, the HFC is preferably C2-C4 HFC, and even more preferably C3 HFC, with penta-fluorinated C3 HFC, such as HFC-245fa, being highly preferred in certain embodiments.

For compositions which include HC co-blowing agents, the HC co-blowing agent (preferably C3, C4 and/or C5 HC) is preferably present in the composition in amounts of from of from about 5% by weight to about 80% by weight of the total blowing agent composition, and even more preferably from about 20% by weight to about 60% by weight of the total blowing agent.

### BLOWING AGENT REFERENCE EXAMPLE

This example demonstrates the performance of Z-HFO-1336mzzm used in combination with hydrocarbon co-blowing agents, and in particular the utility of compositions comprising, or consisting essentially of, Z-HFO-1336mzzm and cyclopentane co-blowing agents in rigid polyurethane insulation foams.

A generic refrigerator appliance-type polyurethane foam formulation (foam forming mixture) is provided. The polyol blend consisted of commercial polyol(s), catalyst(s), surfactant(s), and water. Standard commercial polyurethane processing equipment is used for the foam forming process. A blowing agent combination is formed comprising, or consisting essentially of, Z-HFO-1336mzzm in a concentration of approximately 50 mole percent, and cyclopentane in a concentration of approximately 50 mole percent of the total physical blowing agent. The physical blowing agents can be added individually to the polyol blend or can be pre-blended prior to introduction to the polyol blend.

This example illustrates the physical the thermal conductivity properties of all the resulting foams are suitable for commercial use of these blowing agent combinations.

### FOAMABLE COMPOSITIONS

Also described herein are foamable compositions. As is known to those skilled in the art, foamable compositions generally include one or more components capable of forming foam. As used herein, the term "foam foaming agent" is used to refer to a component, or a combination on components, which are capable of forming a foam structure, preferably a generally cellular foam structure. The foamable compositions described herein include such component(s) and a blowing agent compound, preferably Z-HFO-1336mzzm.

For this use, the amount of the compound Z-HFO-1336mzzm in the composition may be in accordance with the following ranges: from about 1 wt % to about 99 wt %; from about 30 wt % to about 99 wt %; from about 50 wt % to about 99 wt %; from about 75 wt % to about 99 wt %; from about 85 wt % to about 99 wt %; from about 20 wt % to about 80 wt %; from about 90 wt % to about 99 wt %; from about 95 wt % to about 99 wt %; from about 1 wt % to about 20 wt %; from about 1 wt % to about 40 wt %; from about 1 wt % to about 50 wt %; from about 5 wt % to about 20 wt %; from about 5 wt % to about 40 wt %; from about 5 wt % to about 60 wt %; from about 10 wt % to about 80 wt %; from about 10 wt % to about 90 wt %; from about 20 wt % to about 80 wt %; from about 20 wt % to about 90 wt %. Other ranges of amounts are shown in Table 1, and those amounts are likewise applicable for this use of the composition.

The one or more components capable of forming foam may comprise a thermosetting composition capable of forming foam and/or foamable compositions. Examples of thermosetting compositions include polyurethane and polyisocyanurate foam compositions, and also phenolic foam compositions. This reaction and foaming process may be enhanced through the use of various additives such as catalysts and surfactant materials that serve to control and adjust cell size and to stabilize the foam structure during formation. Furthermore, is contemplated that any one or more of the additional components described above with respect to the blowing agent compositions described herein could be incorporated into the foamable composition. In such thermosetting foam embodiments, one or more of the compositions are included as or part of a blowing agent in a foamable composition, or as a part of a two or more part foamable composition, which preferably includes one or more of the components capable of reacting and/or foaming under the proper conditions to form a foam or cellular structure.

The one or more components capable of foaming may comprise thermoplastic materials, particularly thermoplastic polymers and/or resins. Examples of thermoplastic foam components include polyolefins, such as for example monovinyl aromatic compounds of the formula Ar-CHCH2 wherein Ar is an aromatic hydrocarbon radical of the benzene series such as polystyrene (PS). Other examples of suitable polyolefin resins in accordance with the invention include the various ethylene resins including the ethylene homopolymers such as polyethylene and ethylene copolymers, polypropylene (PP) and polyethylene-terephthalate (PET). The thermoplastic foamable composition may be an extrudable composition.

It is contemplated that all presently known and available methods and systems for forming foam are readily adaptable for use in connection with the compositions described herein. For example, the methods described herein generally require incorporating a blowing agent in accordance with the present invention into a foamable or foam forming composition and then foaming the composition, preferably by a step or series of steps which include causing volumetric expansion of the blowing agent in accordance with the present invention.

In general, it is contemplated that the presently used systems and devices for incorporation of blowing agent and for foaming are readily adaptable for use in accordance with the present invention. In fact, it is believed that one advantage of the present invention is the provision of an improved blowing agent which is generally compatible with existing foaming methods and systems.

Thus, it will be appreciated by those skilled in the art that the present invention comprises methods and systems for foaming all types of foams, including thermosetting foams, thermoplastic foams and formed-in-place foams. Thus, one aspect of the present invention is the use of the present blowing agents in connection conventional foaming equipment, such as polyurethane foaming equipment, at conventional processing conditions. The present methods therefore include polyol premix type operations, blending type operations, third stream blowing agent addition, and blowing agent addition at the foam head.

With respect to thermoplastic foams, the preferred methods generally comprise introducing a blowing agent in accordance with the present invention into a thermoplastic material, preferably thermoplastic polymer such as polyolefin, and then subjecting the thermoplastic material to conditions effective to cause foaming. For example, the step of introducing the blowing agent into the thermoplastic material may comprise introducing the blowing agent into a screw extruder containing the thermoplastic, and the step of causing foaming may comprise lowering the pressure on the thermoplastic material and thereby causing expansion of the blowing agent and contributing to the foaming of the material.

It will be appreciated by those skilled in the art, especially in view of the disclosure contained herein, that the order and manner in which the blowing agent of the present invention is formed and/or added to the foamable composition does not generally affect the operability of the present invention. For example, in the case of extrudable foams, it is possible that the various components of the blowing agent, and even the components of the foamable composition, be not be mixed in advance of introduction to the extrusion equipment, or even that the components are not added to the same location in the extrusion equipment. Moreover, the blowing agent can be introduced either directly or as part of a premix, which is then further added to other parts of the foamable composition.

Thus, in certain embodiments it may be desired to introduce one or more components of the blowing agent at first location in the extruder, which is upstream of the place of addition of one or more other components of the blowing agent, with the expectation that the components will come together in the extruder and/or operate more effectively in this manner. Nevertheless, in certain embodiments, two or more components of the blowing agent are combined in advance and introduced together into the foamable composition, either directly or as part of premix which is then further added to other parts of the foamable composition.

### FOAMS

One embodiment of the present invention relates to methods of forming foams, especially panel foams and spray foams, and preferably such foams made from polyurethane and polyisocyanurate. The methods generally comprise providing a blowing agent composition of the present invention, adding (directly or indirectly) the blowing agent composition to a foamable composition, and reacting the foamable composition under the conditions effective to form a foam or cellular structure, as is well known in the art. Any of the methods well known in the art, such as those described in "Polyurethanes Chemistry and Technology," Volumes I and II, Saunders and Frisch, 1962, John Wiley and Sons, New York, N.Y., may be used or adapted for use in accordance with the foam embodiments of the present invention.

For this use, the amount of the compound Z-HFO-1336mzzm in the composition may be in accordance with the following ranges: from about 1 wt % to about 99 wt %; from about 30 wt % to about 99 wt %; from about 50 wt % to about 99 wt %; from about 75 wt % to about 99 wt %; from about 85 wt % to about 99 wt %; from about 20 wt % to about 80 wt %; from about 90 wt % to about 99 wt %; from about 95 wt % to about 99 wt %; from about 1 wt % to about 20 wt %; from about 1 wt % to about 40 wt %; from about 1 wt % to about 50 wt %; from about 5 wt % to about 20 wt %; from about 5 wt % to about 40 wt %; from about 5 wt % to about 60 wt %; from about 10 wt % to about 80 wt %; from about 10 wt % to about 90 wt %; from about 20 wt % to about 80 wt %; from about 20 wt % to about 90 wt %. Other ranges of amounts are shown in Table 1, and those amounts are likewise applicable for this use.

In general, such preferred methods comprise preparing polyurethane or polyisocyanurate foams by combining an isocyanate, a polyol or mixture of polyols, a blowing agent or mixture of blowing agents comprising one or more of the present compositions, and other materials such as catalysts, surfactants, and optionally, flame retardants, colorants, or other additives.

It is convenient in many applications to provide the components for polyurethane or polyisocyanurate foams in pre-blended formulations. Most typically, the foam formulation is pre-blended into two components. The isocyanate and optionally certain surfactants and blowing agents comprise the first component, commonly referred to as the "A" component. The polyol or polyol mixture, surfactant, catalysts, blowing agents, flame retardant, and other isocyanate reactive components comprise the second component, commonly referred to as the "B" component. Accordingly, polyurethane or polyisocyanurate foams are readily prepared by bringing together the A and B side components either by hand mix for small preparations and, preferably, machine mix techniques to form blocks, slabs, laminates, pour-in-place panels and other items, spray applied foams, froths, and the like. Optionally, other ingredients such as fire retardants, colorants, auxiliary blowing agents, and even other polyols can be added as one or more additional streams to the mix head or reaction site. Most preferably, however, they are all incorporated into one B-component as described above.

The present methods and systems also include forming a one component foam, preferably polyurethane foam, containing a blowing agent in accordance with the present invention. In certain preferably embodiments, a portion of the blowing agent is contained in the foam forming agent, preferably by being dissolved in a foam forming agent which is liquid at the pressure within the container, a second portion of the blowing agent is present as a separate gas phase. In such systems, the contained/dissolved blowing agent performs, in large part, to cause the expansion of the foam, and the separate gas phase operates to impart propulsive force to the foam forming agent.

Such one component systems are typically and preferably packaged in a container, such as an aerosol type can, and the blowing agent of the present invention thus preferably provides for expansion of the foam and/or the energy to transport the foam/foamable material from the package, and preferably both. In certain embodiments, such systems and methods comprise charging the package with a fully formulated system (preferably isocyanate/polyol system) and incorporating a gaseous blowing agent in accordance with the present invention into the package, preferably an aerosol type can.

It is contemplated also that in certain embodiments it may be desirable to utilize the present compositions when in the supercritical or near supercritical state as a blowing agent.

The present invention also relates to all foams, including but not limited to closed cell foam, open cell foam, spray foams, panel foams, rigid foam, flexible foam, integral skin and the like, prepared from a polymer foam formulation containing a blowing agent comprising, or consisting essentially of, Z-HFO-1336mzzm, either alone or in combination with one or more other compounds.

Applicants have found that one advantage of the foams, and particularly thermoset foams such as polyurethane foams, in accordance with the present invention is the ability to achieve, preferably in connection with thermoset foam embodiments, exceptional thermal performance, such as can be measured by the K-factor or lambda, particularly and preferably under low temperature conditions, as shown in Figure 1. Although it is contemplated that the present foams, particularly thermoset foams of the present invention, may be used in a wide variety of applications, in certain preferred embodiments the present invention comprises appliance foams in accordance with the present invention, including refrigerator foams, freezer foams, refrigerator/freezer foams, panel foams, and other cold or cryogenic manufacturing applications.

The foams in accordance with the present invention, in certain preferred embodiments, provide one or more exceptional features, characteristics and/or properties, including: thermal insulation efficiency (particularly for thermoset foams), dimensional stability, compressive strength, aging of thermal insulation properties, all in addition to the low ozone depletion potential and low global warming potential associated with many of the preferred blowing agents of the present invention. In certain highly preferred embodiments, the present invention provides thermoset foam, including such foam formed into foam articles, which exhibit improved thermal conductivity relative to foams made using the same blowing agent (or a commonly used blowing agent HFC-245fa) in the same amount but without the compound Z-HFO-1336mzzm.

In other preferred embodiments, the present foams exhibit improved mechanical properties relative to foams produced with blowing agents outside the scope of the present invention. For example, certain preferred embodiments of the present invention provide foams and foam articles having a compressive strength which is superior to, and preferably at least about 10 relative percent, and even more preferably at least about 15 relative percent greater than a foam produced under substantially identical conditions by utilizing a blowing agent consisting of cyclopentane.

Furthermore, it is preferred in certain embodiments that the foams produced in accordance with the present invention have compressive strengths that are on a commercial basis comparable to the compressive strength produced by making a foam under substantially the same conditions except wherein the blowing agent consists of HFC-245fa. In certain preferred embodiments, the foams of the present invention exhibit a compressive strength of at least about 12.5% yield (in the parallel and perpendicular directions), and even more preferably at least about 13% yield in each of said directions.

### SPRAY FOAM EXAMPLES

Spray prepared with Z-HFO-1336mzzm, 1233zd(E), 30/70 mole % blend of 1233zd(E) /1336mzzm and 70/30 mole % blend of 1233zd(E)/1336mzzm had equivalent density. The thermal conductivity data from these foams do not demonstrate the anticipated linear relationship. In fact, foam prepared with a 70/30 mole % 1233zd(E)/1336mzzm and 30/70 mole % 1233zd(E)/1336mzzm have improved k-factors and superior aging to those made with 1233zd(E). This is an unanticipated result.

Spray foams with Z-HFO-1336mzzm, 1233zd(E), 30/70 mole % blend of 1233zd(E)/1336mzzm and 70/30 mole % blend of 1233zd(E)/1336mzzm as blowing agent were prepared as follows. The polyol master batch composition is shown in Table 5 while the generic spray foam formulations with corresponding amounts of blowing agents are listed in Table 6. The foams were prepared with a 3 second pour time and 8 second mix time. The raw materials temperatures were 50 °F polyol/ 70°F MDI.

**Table 5 - Polyol Master Batch Composition**

| Component | Lot Numbers | Php |
|---|---|---|
| Jeffol R-470 x | VC03019501 | 50.00 |
| Terate 4020 | MY4020-18 | 43.75 |
| Diethylene glycol | B11+024 | 6.25 |
| DABCO DC-193 | 0001580875 | 1.25 |
| Dabco DMEA | 258009 | 2.00 |
| Antiblaze AB80 | 122 | 12.50 |
| Water | | 1.25 |
| Total | | 117.00 |

**Table 6 - Spray Foam Formulations**

| | 1233zd(E) | 1233zd(E)/1336mzzm 70/30 mole % | 1233zd(E)/1336mzzm 30/70 mole % | 1336mzzm |
|---|---|---|---|---|
| Mol.% of 1336mzzm | 0 | 30 | 70 | 100 |
| Mol.% of 1233zd(E) | 100 | 70 | 30 | 0 |
| Moles of 1336mzzm | 0 | 0.061 | 0.143 | 0.204 |
| Moles of 1233zd(E) | 0.204 | 0.143 | 0.061 | 0 |
| Polyol Blend | | | | |
| Master Batch | 117.0 | 117.0 | 117.0 | 117.0 |
| 1336mzzm | 0 | 10.0 | 23.5 | 33.5 |
| 1233zd(E) | 26.5 | 18.6 | 7.9 | 0 |
| Total | 143.5 | 145.6 | 148.4 | 150.5 |
| Isocyanate | | | | |
| Lupranate M20 | 137.38 | 137.38 | 137.38 | 137.38 |
| NCO Index | 110 | 110 | 110 | 110 |

### Reactivity

The relationship between cream time, gel time and tack free time are anticipated. They are equivalent for all foams prepared.

**Table 7 - Foam Reactivity**

| | 1233zd(E) | 1233zd(E)/1336mzzm 70/30 mole % | 1233zd(E)/1336mzzm 30/70 mole % | 1336mzzm |
|---|---|---|---|---|
| Reactivity, second | | | | |
| Cream Time | 15 | 15 | 12 | 12 |
| Gel Time | 35 | 34 | 33 | 38 |
| Tack-Free Time | 45 | 45 | 44 | 48 |

### Foam Quality/ Cell size/ Open Cell Content

The foams prepared were well mixed and equivalent in quality. The block density of the foams produced is similar as is the ratio of block to core density. Block density is density of the squared foam prior to sample cutting. Core density is density of the k- factor sample taken from the middle of the sample. This is anticipated since the foams were prepared with equivalent moles of blowing agents.

**Table 8 - Foam Quality: Density/ Cell Size/ Open Cell Content**

| | 1233zd(E) | 1233zd(E)/1336mzzm 70/30 mole % | 1233zd(E)/1336mzzm 30/70 mole % | 1336mzzm |
|---|---|---|---|---|
| Density, lb/ft³ | | | | |
| Foam Density- Block* | 1.8 | 1.78 | 1.81 | 1.79 |
| Foam Density-Core* | 1.83 | 1.71 | 1.77 | 1.82 |
| Ratio Block/ Core | | 1.04 | | |
| Density | 0.98 | | 1.02 | 0.98 |
| Open Cell Content, % | | | | |
| Density, lb/ft³ | | | | |
| Average Cell Size, mm | | | | |
| Average Cell Size | 0.2 | 0.2 | 0.2 | 0.2 |

### Compressive Strength

There is not a linear relationship between blowing agent concentration and perpendicular and parallel compressive strength. However, the variance form the linear relationship is considered minimal.

**Table 9 - Foam Compressive Strength**

| | 1233zd(E) | 1233zd(E)/1336mzzm 70/30 mole % | 1233zd(E)/1336mzzm 30/70 mole % | 1336mzzm |
|---|---|---|---|---|
| Compressive Strength, psi | | | | |
| Perpendicular | 13.163 | 12.869 | 12.132 | 12.38 |
| Parallel | 25.678 | 22.870 | 23.392 | 26.95 |
| Ratio, Perpendicular/Parallel | 0.51 | 0.56 | 0.52 | 0.46 |

### Dimensional Stability

The addition of Z-HFO-1336mzzm to 1233zd(E) foam negatively impacts the dimensional stability of the foam in both cold and hot humid environments. It increases the shrinkage in the cold environment. In the hot humid environment the foams prepared from the blends swell more that the foams prepared from either of the neat compounds.

**Table 10 - Foam Dimensional Stability**

| | 1233zd(E) | 1233zd(E)/1336mzzm 70/30 mole % | 1233zd(E)/1336mzzm 30/70 mole % | 1336mzzm |
|---|---|---|---|---|
| Dimensional Stability, vol.% changed | | | | |
| Cold, -29°C | | | | |
| 1 Day | 0.097 | -0.115 | 0.067 | -0.001 |
| 7 Day | -0.164 | -0.450 | -0.514 | 0.608 |
| 14 Day | -0.134 | -0.347 | -0.133 | -0.108 |

| Hot/Humid, | | | | |
|---|---|---|---|---|
| 70°C/95%R.H. | | | | |
| 1 Day | 5.950 | 4.804 | 7.458 | 5.326 |
| 7 Day | 15.724 | 20.898 | 31.006 | 17.720 |
| 14 Day | 23.598 | 30.314 | 45.523 | 28.501 |

### Thermal Conductivity

The thermal conductivity of foams prepared with these blends are significantly improved over those made with 1233zd(E). Not only are they improved, the improvement is nonlinear in relationship to the amount of Z-HFO-1336mzzm added to the blowing agent blend. It is particularly interesting that the improvement at the low mean temperatures is significant and not 1233zd(E) concentration dependant. In addition, it is notable that the foam prepared from the blends age slower than the 1233zd(E) and the Z-HFO-1336mzzm foams.

**Table 11 - Foam Thermal Conductivity**

| | 1233zd(E) | 1233zd(E)/1336mzzm 70/30 mole % | 1233zd(E)/1336mzzm 30/70 mole % | 1336mzzm |
|---|---|---|---|---|
| Initial | | | | |
| 40 °F | 0.1357 | 0.1328 | 0.1320 | 0.1356 |
| 75 °F | 0.1540 | 0.1485 | 0.1459 | 0.1432 |
| 110 °F | 0.1744 | 0.1667 | 0.1643 | 0.1612 |

| 8 Day | | | | |
|---|---|---|---|---|
| 40 °F | 0.1415 | 0.1360 | 0.1367 | 0.1397 |
| 75 °F | 0.1595 | 0.1527 | 0.1506 | 0.1472 |
| 110 °F | 0.1798 | 0.1719 | 0.1711 | 0.1672 |

| 14 Day | | | | |
|---|---|---|---|---|
| 40 °F | 0.1431 | 0.1371 | 0.1380 | 0.1421 |
| 75 °F | 0.1626 | 0.1558 | 0.1505 | 0.1479 |
| 110 °F | 0.1838 | 0.1772 | 0.1697 | 0.1667 |

Foams prepared with Z-HFO-1336mzzm, 245fa, 30/70 mole % blend of 245fa / Z-HFO-1336mzzm and 70/30 mole % blend of 245fa/Z-HFO-1336mzzm had equivalent density. The dimensional stability and thermal conductivity data form these foams do not demonstrate the anticipated linear relationship. In fact, foam prepared with a 70/30 mole % 245fa/ Z-HFO-1336mzzm have improved k-factors and superior aging to those made with 245fa. This is an unexpected result.

Reference foams were prepared with Z-HFO-1336mzzm, 245fa, 30/70 mole % blend of 245fa/ Z-HFO-1336mzzm and 70/30 mole % blend of 245fa/ Z-HFO-1336mzzm as the blowing agents. The polyol master batch composition is shown above in Table 5 while the generic spray foam formulations with corresponding amounts of blowing agents are listed below in Table 12. The foams were prepared with and 3 second pour time and 8 second mix time. The raw materials temperatures were 50 °F polyol/ 70°F MDI.

**Table 12 - Polyol Master Batch Composition**

| Component | Lot Numbers | php |
|---|---|---|
| Jeffol R-470 x | VC03019501 | 50.00 |
| Terate 4020 | MY4020-18 | 43.75 |
| Diethylene glycol | B11+024 | 6.25 |
| DABCO DC-193 | 0001580875 | 1.25 |
| Dabco DMEA | 258009 | 2.00 |
| Antiblaze AB80 | 122 | 12.50 |
| Water | | 1.25 |
| Total | | 117.00 |

**Table 13 - Generic Spray Foam Formulations**

| | 245fa | 245fa/1336mzzm 70/30 mole % | 245fa/1336mzzm 30/70 mole % | 1336mzzm |
|---|---|---|---|---|
| Mol.% of 1336mzzm | 0 | 30 | 70 | 100 |
| Mol.% of 245fa | 100 | 70 | 30 | 0 |
| Moles of 1336mzzm | 0 | 0.061 | 0.143 | 0.204 |
| Moles of 245fa | 0.204 | 0.143 | 0.061 | 0 |

| Polyol Blend | | | | |
|---|---|---|---|---|
| Master Batch | 117.0 | 117.0 | 117.0 | 117.0 |
| 1336mzzm | 0 | 10.0 | 23.5 | 33.5 |
| 245fa | 27.3 | 19.2 | 8.2 | 0 |
| Total | 144.3 | 146.2 | 148.7 | 150.5 |

| Isocyanate | | | | |
|---|---|---|---|---|
| Lupranate M20 | 137.38 | 137.38 | 137.38 | 137.38 |
| NCO Index | 10 | 110 | 110 | 110 |

### Physical Properties

### Reactivity

The relationship between cream time, gel time and tack free time are anticipated. It would be anticipated that the addition of a high boiler such as Z-HFO-1336mzzm would extend the cream and gel time of the foam system.

**Table 14 - Foam Reactivity**

| | 245fa | 245fa/1336mzzm 70/30 mole % | 245fa/1336mzzm 30/70 mole % | 1336mzzm |
|---|---|---|---|---|
| Reactivity, second | | | | |
| Cream Time | Immediate | 10 | 12 | 12 |
| Gel Time | 29 | 35 | 42 | 38 |
| Tack-Free Time | 43 | 48 | 52 | 48 |

### Foam Quality/ Cell size/ Open Cell Content

The foams prepared were well mixed and equivalent in quality. The block density of the foams produced is similar as is the ratio of block to core density. This is anticipated since the foams were prepared with equivalent moles of blowing agents.

**Table 15 - Foam Quality: Density/ Cell Size/ Open Cell Content**

| | 245fa | 245fa/1336mzzm 70/30 mole % | 245fa/1336mzzm 30/70 mole % | 1336mzzm |
|---|---|---|---|---|
| Density, lb/ft3 | | | | |
| Foam Density- Block | 1.78 | 1.79 | 1.79 | 1.79 |
| Foam Density-Core | 1.75 | 1.94 | 1.86 | 1.82 |
| Ratio Block/ Core | | 0.92 | | |
| Density | 1.02 | | 0.96 | 0.98 |
| Open Cell Content, % | | | | |
| Density, lb/ft3 | | | | |
| Average Cell Size, mm | | | | |
| Average Cell Size | 0.2 | 0.2 | 0.2 | 0.2 |
| Compressive Strength | | | | |

There is a near linear relationship in the perpendicular-to-parallel compressive strength ratios. The ratio decrease with reduction in the use of 245fa.

**Table 16 - Foam Compressive Strength**

| | 245fa | 245fa/1336mzzm 70/30 mole % | 245fa/1336mzzm 30/70 mole % | 1336mzzm |
|---|---|---|---|---|
| Compressive Strength, psi | | | | |
| Perpendicular | 13.24 | 12.32 | 12.48 | 12.38 |
| Parallel | 20.66 | 24.78 | 29.25 | 26.95 |
| Ratio, Perpendicular/Parallel | 0.64 | 0.50 | 0.43 | 0.46 |

### Dimensional Stability

The addition of Z-HFO-1336mzzm to 245fa foam improves the dimensional stability of the foam in both cold and hot humid environments. This is most evident in the hot humid environment. In fact the blends perform better than either of the pure compounds. This is an unexpected result.

**Table 17 - Foam Dimensional Stability**

| | 245fa | 245fa/1336mzzm 70/30 mole % | 245fa/1336mzzm 30/70 mole % | 1336mzzm |
|---|---|---|---|---|
| Dimensional Stability, vol.% changed | | | | |
| Cold, -29°C | | | | |
| 1 Day | -0.066 | -0.245 | -0.236 | -0.001 |
| 7 Day | 1.090 | -0.111 | 0.010 | 0.608 |
| 14 Day | 0.033 | -0.229 | -0.161 | -0.108 |
| | | | | |

| Hot/Humid, | | | | |
|---|---|---|---|---|
| 70°C/95%R.H. | | | | |
| 1 Day | 7.388 | 3.657 | 4.899 | 5.326 |
| 7 Day | 21.617 | 14.060 | 16.385 | 17.720 |
| 14 Day | 30.616 | 22.013 | 26.563 | 28.501 |
| Thermal Conductivity | | | | |

Initially, foam produced with Z-HFO-1336mzzm and the 70/30 mole % Z-HFO-1336mzzm/245fa blend show the "hockey stick" curve shape traditionally found with high boiling blowing agents. This is attributed to the condensation of the blowing agent in the foam matrix at temperatures below the boiling point of the blowing agent. It is unanticipated that the 30/70 mole % Z-HFO-1336mzzm/245fa blend does not show the same curve shape since this is not an azeotropic composition. In addition the thermal conductivity of foams prepared with this blend is equivalent or slightly improved over those made with 245fa. The foam prepared of the 70/30 mole % 245fa/ Z-HFO-1336mzzm blend age slower than the 245fa and the Z-HFO-1336mzzm foams.

**Table 18 - Foam Thermal Conductivity**

| | 245fa | 245fa/1336mzzm 70/30 mole % | 245fa/1336mzzm 30/70 mole % | 1336mzzm |
|---|---|---|---|---|
| Initial | | | | |
| 40 °F | 0.1318 | 0.1319 | 0.1347 | 0.1356 |
| 75 °F | 0.1481 | 0.1476 | 0.1452 | 0.1432 |
| 110 °F | 0.1660 | 0.1663 | 0.1629 | 0.1612 |

| 8 Day | | | | |
|---|---|---|---|---|
| 40 °F | 0.1381 | 0.1365 | 0.1380 | 0.1397 |
| 75 °F | 0.1555 | 0.1508 | 0.1493 | 0.1472 |
| 110 °F | 0.1742 | 0.1695 | 0.1692 | 0.1672 |

| 14 Day | | | | |
|---|---|---|---|---|
| 40 °F | 0.1409 | 0.1376 | 0.1391 | 0.1421 |
| 75 °F | 0.1585 | 0.1528 | 0.1524 | 0.1479 |
| 110 °F | 0.1774 | 0.1716 | 0.1740 | 0.1667 |

While the present invention has been particularly shown and described with reference to preferred embodiments, it will be readily appreciated by those of ordinary skill in the art that various changes and modifications may be made without departing from the scope of the invention.

## Claims

1. A mixture comprising the compound cis-1,1,1,4,4,4-hexafluoro-2-butene (Z-HFO-1336mzzm) and HFO-1233zd for use as a polyol premix composition; wherein said composition comprises from 30 wt.% to 70 wt.% of said HFO-1233zd.

2. A mixture comprising the compound cis-1,1,1,4,4,4-hexafluoro-2-butene (Z-HFO-1336mzzm) and HFO-1233zd for use as a blowing agent composition; wherein said composition comprises from 30 wt.% to 70 wt.% of said HFO-1233zd.

## Patentansprüche

1. Mischung, umfassend die Verbindung cis-1,1,1,4,4,4-Hexafluor-2-buten (Z-HFO-1336mzzm) und HFO-1233zd, zur Verwendung als Polyol-Premixzusammensetzung; wobei die Zusammensetzung 30 Gew.-% bis 70 Gew.-% des HFO-1233zd umfasst.

2. Mischung, umfassend die Verbindung cis-1,1,1,4,4,4-Hexafluor-2-buten (Z-HFO-1336mzzm) und HFO-1233zd, zur Verwendung als Treibmittelzusammensetzung; wobei die Zusammensetzung 30 Gew.-% bis 70 Gew.-% des HFO-1233zd umfasst.

## Revendications

1. Mélange comprenant le composé cis-1,1,1,4,4,4-hexafluoro-2-butène (Z-HFO-1336mzzm) et HFO-1233zd pour une utilisation en tant que composition de prémélange de polyol ; ladite composition comprenant 30 % en poids à 70 % en poids dudit HFO-1233zd.

2. Mélange comprenant le composé cis-1,1,1,4,4,4-hexafluoro-2-butène (Z-HFO-1336mzzm) et HFO-1233zd pour une utilisation en tant que composition d'agent de gonflement ; ladite composition comprenant 30 % en poids à 70 % en poids dudit HFO-1233zd.
